# EUROPÄISCHE PATENTSCHRIFT

(11) **EP 0 998 237 B1**
(45) Veröffentlichungstag und Bekanntmachung des Hinweises auf die Patenterteilung: **01.10.2003**
(21) Anmeldenummer: 98943734.8
(22) Anmeldetag: 17.07.1998
(51) Int. Cl.: A61B 17/66

(54) **INTRAORALER DISTRAKTOR FÜR KALLUSDISTRAKTION IM UNTERKIEFER**
INTRAORAL DISTRACTOR FOR CALLUS DISTRACTION IN THE LOWER JAW
SEPARATEUR INTRA-BUCCAL POUR LA SEPARATION DE CALS DANS LE MAXILLAIRE INFERIEUR

(30) Priorität: 21.07.1997 CH 176997; 30.06.1998 CH 138398
(43) Veröffentlichungstag der Anmeldung: 10.05.2000
(73) Patentinhaber: Minoretti, Roger, 8006 Zürich (CH); Minoretti, André, 8605 Gutenswil (CH)
(72) Erfinder: Minoretti, Roger, 8006 Zürich (CH); Minoretti, André, 8605 Gutenswil (CH)
(74) Vertreter: Grimm, Ekkehard, Dipl.-Phys.
(86) Internationale Anmeldenummer: EP9804421
(87) Internationale Veröffentlichungsnummer: WO99004715

(56) Entgegenhaltungen:
- WO-A-97/14367
- DE-A- 19 537 023
- DE-U- 29 501 880
- US-A- 5 540 687

## Beschreibung

Die vorliegende Erfindung betrifft einen intraoralen Distraktor für Kallusdistraktion im Unterkiefer eines Menschen mit einem proximalen (posterioren) und einem distalen (anterioren) Befestigungsteil und mit einem ersten Verstellantrieb zur linearen Veränderung des Abstands zwischen den beiden Befestigungsteilen zur Verlängerung des corpus mandibulae oder des ramus mandibulae im Bereich einer Osteotomie.

Ein solcher intraoraler Distraktor ist aus der DE-A1-195 37 023 bekannt.

Bei einem Distraktor für Kallusdistraktion im Unterkiefer eines Menschen handelt es sich um eine kombiniert chirurgisch-kieferorthopädische Apperatur, um mittels Extension von Kallus-Okklusionsstörungen, Malformationen, Asymmetrien des Unterkiefers und Mißverhältnisse von Ober- zu Unterkiefer zu beheben, die angeboren oder erworben sein können, letzteres beispielsweise durch einen Unfall.

Solche Distraktoren ermöglichen, den aufsteigenden Ast (ramus mandibulae) oder den horizontalen Teil (corpus mandibulae) des Unterkiefers, im Bereich einer Osteotomie, das bedeutet im Bereich einer Durchtrennung des Knochens, linear zu verlängern. Darüberhinaus ist es möglich, durch Veränderung des Kieferwinkels die Okklusionsebene der Unterkieferzähne zu schwenken und damit die Okklusionsebene den Oberkieferzähnen anzupassen; hierzu werden ramus mandibulae und corpus mandibulae im Bereich der Osteotomie um einen Winkel zueinander verschwenkt.

Es muß zwischen intraoralen Distraktoren und extraoralen Distraktoren für Kallusdistraktion im Unterkiefer eines Menschen unterschieden werden. Beide Distraktoren besitzen Befestigungsteile, die an dem Unterkiefer, beidseitig der vorzunehmenden Osteotomie, befestigt werden. Bei einem extraoralen Distraktor werden dann von diesen Befestigungselementen aus Fixierungsstifte durch die Haut hindurch nach außen (extraoral) geführt und mit Verstellelementen, beispielsweise Gewindestangen und Gewindehülsen, verbunden. Der Vorteil solcher extraoralen Distraktoren ist derjenige, daß die Verstellelemente gut von der Außenseite zugänglich sind. Allerdings haben sie den Nachteil, daß die Fixierungstifte durch die Haut hindurchgeführt werden, was, je nach Größe der Wunden in der Haut, Narben hinterläßt.

Intraorale Distraktoren werden einschließlich der Verstellelemente innerhalb der Mundhöhle positioniert. Für solche Distraktoren sind demzufolge keine Durchführungen von Stiften oder Halteteilen durch die Haut erforderlich. Allerdings ist der zur Verfügung stehende Raum innerhalb der Mundhöhle begrenzt, so daß es schwierig ist, geeignete Verstellantriebe dort gut zugänglich anzuordnen.

Die Distraktionsvorrichtung, wie sie aus der DE- A1-195 37 023 bekannt ist, umfaßt kleine zueinander verstellbare Halterungen bzw. Befestigungsteile, wobei dem einen Befestigungsteil eine Hülse zugeordnet ist, während dem anderen Befestigungsteil eine Gewindestange zugeordnet ist, die in der Hülse geführt ist. Die Gewindestange kann in der Hülse verdreht werden, so daß unter einem solchen Verdrehen der Abstand zwischen den beiden Befestigungsteilen kontiunierlich, linear verstellbar ist.

Weiterhin ist bei der Anordnung von Distraktoren am Unterkiefer zu beachten, daß der im sogenannten canalis mandibulae verlaufende nervus alveolaris inferior, welcher am foramen mentale im Bereich des corpus mandibulae austritt und die Unterlippe sensibel versorgt, nicht durch Befestigungsteile, die am Unterkiefer verschaubt werden, beschädigt wird oder beschädigt werden könnte.

Eine weitere Vorrichtung zur Fixierung von Knochensegmenten, die auch im Bereich des Unterkiefers eingesetzt werden kann, ist aus der DE-A1 195 03 609 bekannt. Hierbei handelt es sich um eine Anordnung mit einem mehrteiligen Haltestrang, der einzelne Halteelemente aufweist, die jeweils an einem Zahnstangenstrang angeordnet sind. Die jeweiligen Halteelemente sind mit Haltestiften am Unterkiefer, durch die Haut hindurchführend, verankert. Zwischen jeweils zwei Halteelementen kann eine Osteotomie erfolgen. Die Veränderung der Lage der jeweiligen Knochenhälften beidseitig der Osteotomie erfolgt dann über die Halteelemente, die an der Zahnstange linear verschoben werden. Eine solche Anordnung ist nur als extraoraler Distraktor einsetzbar.

Eine weitere Distraktionsvorrichtung ist aus der US-PS 5,203,783 bekannt. Diese Anordnung umfaßt ein Antriebsteil, mit einem Gehäuse, aus dem ein kolbenartiges Teil herausschiebbar ist. An dem Ende des kolbenartigen Teils ist eine Scheibe angeordnet, die auf der einen Seite über eine Stange mit dem Gehäuse verbunden ist, während sie an der gegenüberliegenden Seite ein mit einem Knochen zu verbindendes Halteelement besitzt. Unter Verstellung, d.h. Verlängerung oder Verkürzung, des kolbenartigen Teils wird die Scheibe gedreht, so daß mit dieser Drehung auch eine winkelmäßige Verschwenkung des am Knochen befestigten Halteelements erfolgt. Diese Anordnung ist von einer großen Bauform und nicht als Distraktor für Kallusdistraktion im Unterkiefer, insbesondere auch nicht als intraoraler Distraktor, geeignet.

Ausgehend von dem vorstehend beschriebenen Stand der Technik liegt der vorliegenden Erfindung die Aufgabe zugrunde, einen intraoralen Distraktor für Kallusdistraktion im Unterkiefer der eingangs angegebenen Art derart weiterzubilden, daß er, obwohl als intraoraler Distraktor einsetzbar, nicht nur eine lineare Distraktion von corpus mandibulae oder ramus mandibulae im Bereich einer Osteotomie ermöglicht, sondern auch eine winkelmäßige Umorientierung von corpus mandibulae und ramus mandibulae zuläßt und auch die Möglichkeit bietet, eine Verlängerung von ramus mandibulae, corpus mandibulae und eine winkelmäßige Verstellung von ramus mandibulae zu corpus mandibulae vorzunehmen.

Diese Aufgabe wird bei einem Distraktor mit den eingangs genannten Merkmalen gemäß dem kennzeichnenden Teil des Anspruchs 1 dadurch gelöst, daß ein Distraktionselement mit einem Gelenk vorgesehen ist, dessen Gelenkachse so orientiert ist, daß es eine Veränderung der winkelmäßigen Zuordnung von corpus mandibulae und ramus mandibulae zueinander im Bereich der Osteotomie ermöglicht, und wobei das Distraktionselement derart mit den Befestigungsteilen verbunden ist, daß es außerhalb des Gewebes im cavum oris und bukkal der Zahnreihe im Vestibulum liegt. Dieser Distraktor für den Einsatz im Unterkiefer ermöglicht, sowohl den aufsteigenden Ast (ramus mandibulae) als auch den horizontalen Teil des Unterkiefers (corpus mandibulae) durch Kallusdistraktion einzeln oder in Kombination zu verlängern und zusätzlich, was wesentlich ist, aufgrund des Gelenks den Kieferwinkel zu verändern. Durch eine solche Veränderung des Kieferwinkels kann die Okklusionsebene der Unterkieferzähne geschwenkt und der Okklusionsebene der Oberkieferzähne angepaßt werden. Vorzugsweise wird hierbei die Osteotomie durch den angulus mandibulae ungefähr in der Winkelhalbierenden gelegt. Im Bereich dieser Osteotomie ist dann die Möglichkeit gegeben, den aufsteigenden und/oder den horizontalen Ast des Unterkiefers zu verlängern und gleichzeitig unter Veränderung der Winkelstellung des Distraktors über das Gelenk die winkelmäßige Zuordnung zwischen horizontalem Ast und aufsteigendem Ast den Gegebenheiten im Oberkiefer, d.h. der Okklusionsebene und der Okklusion, anzupassen. Eine weitere, wesentliche Maßnahme dafür, daß der Distraktor, trotz des vorgesehenen Gelenks und eines weiteren Verstellantriebs für die Verlängerung der Kieferäste, intraoral einsetzbar ist, ist diejenige, daß das Distraktionselement außerhalb des Gewebes im cavum oris und bukkal der Zahnreihe im Vestibulum liegt. Eine solche Vorrichtung ist darüberhinaus sowohl für die rechte als auch die linke Unterkieferseite geeignet, d.h. in einem spiegelsymmetrischen Aufbau der Distraktoren zueinander, einzusetzen und diese unabhängig voneinander zu manipulieren. Der Einsatz einer solchen Anordnung beidseitig oder nur einseitig hängt von der Art der Unterkieferfehlstellung ab.

Der Distraktor wird, unabhängig der auszuführenden Korrekturbewegung, proximal (posterior) der Osteotomie mit Schrauben am ramus mandibulae und, in einer ersten Varianten, distal (anterior) der Osteotomie, ebenfalls mit Schrauben, in der Kinnregion apikal der ipsilateralen Frontzähne, oder, in einer zweiten Varianten, distal (anterior) der Osteotomie, ebenfalls mit Schrauben, am corpus mandibulae posterior des foramen mentale befestigt.

Mit dem vorstehend angegebenen Aufbau erfolgt die vorzunehmende Osteotomie von enoral im Kieferwinkel (angulus mandibulae) unter absoluter Schonung des nervus alveolaris inferior, indem bukkal und lingual eine Kortikotomie durchgeführt und die spongiosa manuell frakturiert wird. Über einen extraoralen, transbukkalen Zugang mittels einer kleinen Hautinzision wird der Unterrand des Unterkiefers im Kieferwinkelbereich mit der Fräse kortikotomiert und mit einem Bohrer werden die Bohrlöcher für die Schrauben am aufsteigenden Ast gebohrt. Die Schrauben werden ebenfalls transbukkal eingebracht.

In der vorstehend erwähnten ersten Variante erfolgt in der Kinnregion der Zugang von enoral über eine Schnittführung in der beweglichen Schleimhaut im Vestibulum in einem Abstand von ungefähr 8 mm von der Grenze zur angewachsenen Gingiva der Unterkieferfront in der Region des Eckzahns. Der Austritt des nervus mentalis aus dem foramen mentale wird dargestellt. Bei der zweiten Varianten erfolgt die Inzision von enoral im bukkalen Vestibulum in der beweglichen Schleimhaut, das Periost wird am corpus mandibulae partiell abgeschoben, der nervus mentalis am foramen mentale dargestellt und der distale (anteriore) Arm, der in einer solchen Ausführungsform vorzugsweise senkrecht verläuft und bis zum Unterkieferrand reicht, mit Schrauben posterior vom foramen mentale fixiert. Der proximale (posteriore) am Knochen befestigte Arm tritt am Vorderrand des ramus mandibulae durch die Schleimhaut ins bukkale Vestibulum und der distale (anteriore) Arm, bei der ersten Varianten, in der Umschlagsfalte des Vestibulums der Unterkieferfront ein; in der zweiten Variante tritt der distale (anteriore) Arm in der Umschlagsfalte des bukkalen Vestibulums in das cavum oris ein.

Das zentrale Bauteil des Distraktors ist das Distraktionselement mit dem darin vorgesehenen Gelenk. Das Distraktionselement ist außerhalb der Gewebe bukkal der Zahnreihen im Vestibulum der Mundhöhle angeordnet. Vorzugsweise wird diesem Distraktionselement auch der erste Verstellantrieb als Teil davon zugeordnet. Auf diese Art und Weise ist sowohl die winkelmäßige Verschwenkung über die beiden Kieferäste beidseitig der Osteotomie als auch die lineare Veränderung des Abstands zwischen den beiden Befestigungsteilen zur Verlängerung des corpus mandibulae und/oder des ramus mandibulae. Ferner ist das Distraktionselement des intraoralen Distraktors zwischen den Befestigungsteilen lösbar im Bereich von Trennstellen gehalten. Hierdurch kann dieses Bauteil herausgenommen werden, um es durch eine veränderte Variante gegebenfalls den Gegebenheiten im Unterkiefer im Rahmen der Behandlung des Patienten anpassen zu können; darüberhinaus ist auch die Möglichkeit gegeben, während des operativen Eingriffs zunächst die Befestigungsteile fest zu positionieren und den Wundverschluß vorzunehmen, um anschließend dann das Distraktionselement zwischen die Befestigungsteile einzusetzen.

Während in vielen Fällen ein linearer Verstellantrieb, wie er vorstehend erwähnt ist, ausreichend ist, um den Abstand zwischen den beiden Befestigungsteilen linear zu verändern, kann es in besonderen Fällen, nämlich dann, wenn neben dem corpus mandibulae auch der ramus mandibulae verlängert werden muß von Vorteil sein, einen zweiten, linearen Verstellantrieb vorzusehen, mit dem eine lineare Veränderung des Abstands zwischen den beiden Befestigungsteilen in einer Richtung senkrecht zu der linearen Veränderung des ersten Verstellantriebs möglich ist. Mit dieser doppelten, linearen Verschiebbarkeit, zusätzlich zu der Winkelverschwenkung, ist praktisch jede räumliche Anpassung der Okklusionsebene des Unterkiefers zu der Okklusionsebene der Zähne im Oberkiefer anpassbar. In einer solchen Anordnung sollte wiederum, in einer bevorzugten, konstruktiven Ausbildung des Distraktors, der zweite Verstellantrieb dem Distraktionselement zugeordnet werden, so daß alle Verstellmöglichkeiten zentral an dem Distraktionselement, und damit gut zugänglich in der Mundhöhle, vorgenommen werden können.

Die Reihenfolge der Anordnung der einzelnen Verstellantriebe innerhalb des Distraktionselements kann gegebenenfalls geändert werden.

Falls ein erster und ein zweiter Verstellantrieb vorgesehen werden, sollten diese bevorzugt so aufgebaut werden, daß der erste Verstellantrieb im wesentlichen eine Verlängerung des corpus mandibulae vornimmt, während der zweite Verstellantrieb im wesentlichen eine Verlängerung des ramus mandibulae vornimmt.

Um die linearen Verstellmöglichkeiten konstruktiv einfach aufbauen zu können, weist in einer Ausführungsform des Distraktors das Distraktionselement einen Schenkel auf, der dem am ramus mandibulae verschraubten Befestigungsteil zugeordnet ist, und ein Schenkel, der dem dem corpus mandibulae zugeordneten Befestigungsteil zugeordnet ist. In einer solchen Anordnung können weiterhin die beiden Schenkel mit ihren freien Enden, in Richtung der Erstreckung des corpus mandibulae gesehen, überlappen, so daß über das freie Stirnende des einen überlappenden Teils, das zu den Frontzähnen hinweist, eine entsprechende Verstellmöglichkeit vorgesehen werden kann, beispielsweise eine Gewindespindel.

Falls der Distraktor mit einem ersten und einem zweiten Verstellantrieb versehen wird, sollte der erste Verstellantrieb dem Schenkel des Distraktionselements zugeordnet werden, der Teil des dem corpus mandibulae zugeordneten Befestigungsteils ist, während der zweite Verstellantrieb eine Verlängerung des ramus mandibulae vornimmt.

Eine einfache, raumsparende Verstellmöglichkeit ergibt sich dadurch, daß der Schenkel, der zu dem Befestigungsteil gehört, das dem corpus mandibulae zugeordnet ist, in zwei Schenkelteile unterteilt wird und die beiden Schenkelteile mittels einer darin geführten Drehwelle in ihrem Abstand zueinander linear verstellt werden. Eine solche Drehwelle ist wiederum gut von der Seite der Frontzähne aus zugänglich.

Weiterhin sollte das dem ramus mandibulae zugeordnete Befestigungsteil so ausgelegt, d.h. geformt, sein, daß es auf der bukkalen bzw. lateralen Seite des ramus mandibulae befestigbar ist.

Da der transversale Abstand zwischen den Kieferköpfchen während der Distraktion nicht verändert werden darf, sollte(n) die Richtung(en) der linearen Veränderung(en) parallel zur Sagittalebene und die Achse der Winkelveränderung senkrecht zur Sagittalabene angeordnet sein. Außerdem sollte, im Hinblick auf eine Ausrichtung der Kieferköpfchen, die Gelenkachse senkrecht zur Sagittalebene ausgerichtet sein.

Um einen kompakten Aufbau zu erzielen, allerdings mit der Möglichkeit, eine feinfühlige Verstellung vornehmen zu können, sollte der erste und/oder der zweite Verstellantrieb durch eine Zahnstange und ein darin eingreifendes Schneckenzahnrad ausgebildet sein. Mit einer solchen Anordnung kann erreicht werden, daß eine Drehung des Schneckenzahnrads von der Frontseite möglich ist, während die Verstellung der Zahnstange in einer Richtung senkrecht dazu erfolgt, d.h. es ist mit einem solchen Aufbau unter Einsatz eines Schneckenzahnrads eine Umlenkung um einen bestimmten Winkel zwischen Antriebs-Richtung und Abtriebs-Richtung möglich.

Ein weiterer konstruktiver Aufbau für die Veränderung der winkelmäßigen Zuordnung von ramus mandibulae und corpus mandibulae ist mittels eines Zahnradsegments mit Schrägverzahnung und einem darin eingreifenden Schneckenzahnrad möglich, wiederum mit den Vorteilen, wie sie vorstehend erläutert sind. Unter Verwendung eines solchen Schneckenzahnrads sollte das Zahnradsegment an dem dem ramus mandibulae zugeordneten Befestigungsteil angeordnet sein, während das Schnekkenzahnrad an dem dem corpus mandibulae zugeordneten Befestigungsteil angeordnet ist.

In Verbindung mit einem Distraktionselement, das zwischen den Befestigungsteilen lösbar im Bereich von Trennstellen gehalten ist, wie dies weiter vorstehend erläutert ist, sollten die Trennstellen zwischen dem Distraktionselement und dem jeweiligen Befestigungsteil jeweils mittels einer linearen Führung gebildet sein, wobei die Führungsrichtung parallel zur Sagittalebene und etwa senkrecht zur Okklusionsebene des Unterkiefers verläuft. Hierbei kann ein konstruktiv einfacher Aufbau dadurch erzielt werden, daß die lineare Führung an der Trennstelle zu dem corpus mandibulae hin durch eine in eine Nut-Führung eingreifende Lasche gebildet ist. Alternativ hierzu ist ein konstruktiv einfacher Aufbau auch dadurch möglich, daß die lineare Führung an der Trennstelle zu dem ramus mandibulae hin durch eine auf eine Rundstab-Führung aufgesetzte Hülse oder Klaue gebildet ist. Die Verbindung zwischen der Rundstab-Führung und der darauf aufgesetzten Hülse oder Klaue sollte darüberhinaus so ausgebildet sein, daß das Distraktionselement durch eine Verschwenkung um die durch die Rundstab-Führung gebildete Achse parallel zu der Sagittalebene ausrichtbar ist. Weiterhin ermöglicht ein solcher Aufbau, daß die Hülse entlang der Stabführung in Richtung deren Achse zur höhenmäßigen Ausrichtung verstellbar ist, wobei eine entsprechende Verstelleinrichtung vorgesehen sein sollte, um die Hülse an der Rundstab-Führung in der gewünschten Stellung zu fixieren. Eine solche Rundstab-Führung sollteTeil des Befestigungsteils sein.

Weitere Einzelheiten und Merkmale der vorliegenden Erfindung ergeben sich aus der nachfolgenden Beschreibung von Ausführungsbeispielen anhand der Zeichnungen. In der Zeichnung zeigt
- Figur 1: eine Draufsicht auf einen Unterkiefer mit zwei spiegelsymmetrisch zueinander aufgebauten intraoralen Distraktoren gemäß einer ersten Ausführungsform,
- Figur 2: eine Seitenansicht des Distraktors der Figur 1 in Richtung des Sichtpfeils II in Figur 1,
- Figur 3: eine zweite Ausführungsform eines intraoralen Distraktors in einer Draufsicht,
- Figur 4: eine Seitenansicht des Distraktors der Figur 3 in Richtung des Sichtpfeils IV in Figur 3,
- Figur 5: eine dritte Ausführungsform eines intraoralen Distraktors in einer Draufsicht,
- Figur 6: eine Seitenansicht des Distraktors der Figur 5 in Richtung des Sichtpfeils VI in Figur 5,
- Figur 7: eine schematische Darstellung einer vierten Ausführungsform eines Distraktors mit einem Schneckenantrieb,
- Figur 8: eine Darstellung der Wirkungslinien und der Befestigungspunkte des Distraktors der Figur 7,
- Figur 9A: eine Seitenansicht des vorderen Teils des Distraktorelements der Figur 7,
- Figur 9B: eine Ansicht auf das Bauteil der Figur 9A aus Richtung des Sichtpfeils IXB in Figur 9A,
- Figur 10A: eine Ansicht des Gelenkteils des Distraktionselement der Figur 7 aus Richtung des Sichtpfeils VIIIA,
- Figur 10B: eine Ansicht des Teils der Figur 10A aus Richtung des Sichtpfeils XB in Fig. 10A und
- Figur 10C: eine Ansicht auf das Teil aus Richtung des Sichtpfeils IX der Figur 10A.

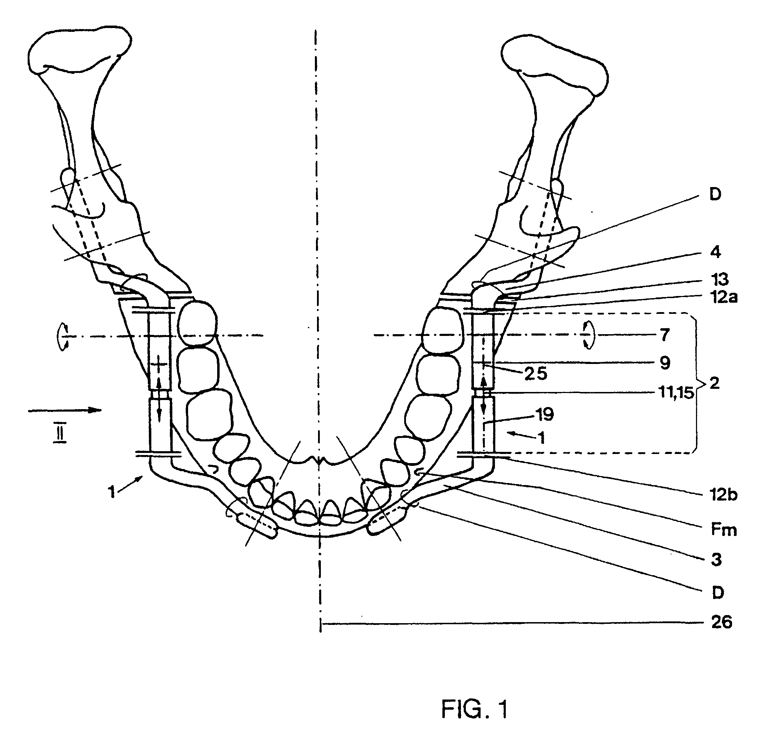

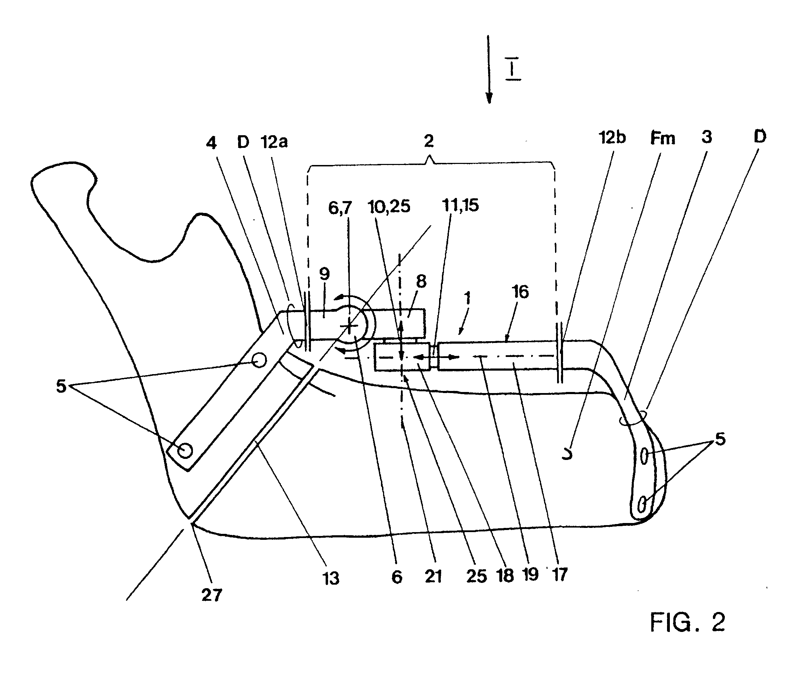

Der Distraktor 1 weist in einer ersten Ausführungsform, wie sie in den Figuren 1 und 2 dargestellt ist, zwischen zwei Befestigungsteilen 3 und 4 ein Distraktionselement 2 auf. Das Befestigungselement 3, d.h. der distale (anteriore) Arm, tritt in der Umschlagsfalte des Vestibulums der Unterkieferfront in das cavum oris ein. Das andere Befestigungsteil 4 tritt am Vorderrand des ramus mandibulae durch die Schleimheit (Mucosa) ins bukkale Vestibulum an der mit D bezeichneten Stelle ein. Die jeweiligen Befestigungsteile 3, 4 sind der Wölbung des corpus mandibulae bzw. des ramus mandibulae angepaßt und weisen jeweils zwei, gegebenenfalls auch drei oder vier, Befestigungslöcher 5 auf, wie in der Seitenansicht der Figur 2 zu sehen ist.

Zentrales Teil des Distraktionselements 2 ist ein Gelenk 6, dessen Gelenkachse mit dem Bezugszeichen 7 bezeichnet ist. Beidseitig des Gelenks 6 erstrecken sich zwei Schenkel 8, 9, wobei der eine Schenkel 8 des Distraktionselements 2 mit dem Befestigungsteil 3 verbunden ist, während der Schenkel 9 mit dem Befestigungsteil 4 verbunden ist. Das Distraktionselement 2 befindet sich, wie insbesondere der Figur 2 zu entnehmen ist, außerhalb des Gewebes im bukkalen Vestibulum lateral der Zahnreihen und ermöglicht eine Distraktion in cranio-caudaler Richtung, wie durch den Doppelpfeil 10 angedeutet ist, und eine Verschiebung in posterio-anteriorer Richtung, wie durch den Doppelpfeil 11 angedeutet ist. Weiterhin sind im Übergangsbereich zwischen den Befestigungsteilen 3 und 4 und dem Distraktionselement 2 bzw. dessen Schenkeln 8 und 9 Trennstellen 12a, 12b vorgesehen, so daß das Distraktionselement 2 an diesen Trennstellen 12a und 12b, über geeignete, dort angeordnete Verbindungsmechanismen, von dem proximalen (posterioren) Befestigungsteil 4 und dem anterioren Befestigungsteil 3 gelöst und entfernt bzw. wieder eingefügt werden kann. Dies erleichtert intraoperativ die Naht der Mucosa im Vestibulum und ermöglicht auch, während der Behandlung den Distraktionsmechanismus auszutauschen, falls dies die Behandlungsmaßnahmen erfordern sollten.

Bei extrem langer Distraktionsstrecke könnte es vorkommen, daß der im Distraktionselement zur Verfügung stehende Distraktionsweg ausgenutzt wurde, obwohl klinisch betrachtet eine weitere Distraktion nötig wäre. In einem solchen Fall könnte dann das Distraktionselement 2 gegen ein solches ausgetauscht werden, das einen längeren Distraktionsweg ermöglicht. Weiterhin ermöglicht die Herausnahme des Distraktionselements 2, daß dann, wenn das Distraktionsziel erreicht ist, die Distraktionsstrecke bzw. die über das Gelenk 6 vorgenommene Winkeländerung abgelesen und für die Zeit der Haltephase, d.h. auch bis zur endgültigen Ausheilung, ein der Dimension entsprechender Platzhalter anstelle des Distraktionselements 2 eingesetzt werden kann. Ein solcher Platzhalter kann sehr klein dimensioniert werden, so daß der Austausch des Distraktionselements 2 gegen einen solchen Platzhalter den Tragekomfort für den Patienten erleichtert und verbessert.

Wie anhand der Figur 2 zu sehen ist, ist das Gelenk 6 mit seiner Gelenkachse 7 in Verlängerung der vorzunehmenden Osteotomie 13 bzw. in die Ebene der vorzunehmenden Osteotomie gelegt, wobei die Osteotomie vorzugsweise durch den angulus mandibulae 27 ungefähr in der Winkelhalbierenden gelegt wird. Dadurch, daß das Gelenk 6 in der Ebene der Osteotomie 13 liegt, kann, durch eine winkelmäßige Verstellung der Schenkel 8 und 9 zueinander in Richtung des Doppelpfeils 14 eine gleichmäßige Winkelverstellung von ramus mandibulae zu corpus mandibulae erfolgen. Eine zusätzliche Korrektur kann gleichzeitig zu der Winkeländerung oder in zusätzlichen Schritten, durch die lineare Verlängerung oder Verkürzung des dem corpus mandibulae zugeordneten Schenkels 8 in Richtung des Doppelpfeils 11 oder aber durch eine Verschiebung senkrecht zu der Okklusionsebene bzw. senkrecht zu dem ersten Verschiebeweg in Richtung des Doppelpfeils 10 erfolgen.

Für eine solche Verlängerung oder Verkürzung in Richtung des Doppelpfeils 11 ist ein erster Verstellantrieb 15 vorgesehen, indem der freie Schenkel 16 des distalen (anterioren) Befestigungsteils 3, der mit seiner Längserstreckung etwa parallel zu der Okklusionsebene verläuft, in zwei Abschnitte 17 und 18 unterteilt ist, wobei diese beiden Abschnitte 17 und 18 durch eine Gewindespindel, deren Achse durch die strichpunktierte Linie 19 angedeutet ist, rotationsgesichert miteinander verbunden sind. Die Gewindespindel 19 ist bis zu dem vorderen Ende 20 des Abschnitts 17 geführt und ist an dieser Stelle über eine nicht näher dargestellte Öffnung für ein Werkzeug zugänglich. Hierzu kann in der Stirnseite dieser Gewindespindel ein Schlitz, ein Kreuzschlitz oder eine Inbusöffnung oder ein entsprechender Dorn, vorgesehen sein, so daß ein entsprechendes Werkzeug angesetzt werden kann. Mit einem solchen Werkzeug kann dann die Gewindespindel gedreht werden, um den Abstand zwischen dem Abschnitt 18 und dem Abschnitt 19 im Bereich des Doppelpfeils 11 zu verändern und damit im Bereich der Osteotomie der corpus mandibulae zu dem ramus mandibulae in Richtung des Doppelpfeils 11 zu verschieben.

Um den corpus mandibulae in Richtung des Doppelpfeils 10 zu verschieben, d.h. in der cranio-candalen Richtung (oder etwa senkrecht zur Okklusionsebene), ist der Abschnitt 18 des Schenkels 16 des anterioren Befestigungsteils 3 unterhalb des Schenkels 8 des Distraktionselements 2 positioniert. In dieser Ausrichtung sind der Schenkel 8 und der Abschnitt 18 über eine weitere Gewindespindel, deren Achse mit der strichpunktierten Linie 21 angedeutet ist, einen zweiten Verstellantrieb 25 bildend, verbunden. Unter Drehung der Gewindespindel wiederum mit einem geeigneten Werkzeug, an deren Stirnende (z.B. mittels Kegelradverbindung), das an der Oberseite des Schenkels 8 zugänglich ist, eingreifend, kann die Gewindespindel in Richtung des Doppelpfeils 10, von der Mundhöhle aus gut zugänglich, verstellt werden.

Wie die erste Ausführungsform des intraoralen Distraktors, dargestellt in den Figuren 1 und 2, zeigt, ist damit, trotz eines mechanisch einfachen Aufbaus, eine Veränderung der Orientierung von ramus mandibulae und corpus mandibulae zueinander mit drei Freiheitsgraden, d.h. in Richtung der Doppelpfeile 10 und 11 sowie um die Gelenkachse 7, möglich, so daß die Okklusionsebene des Unterkiefers zu der Okklusionsebene des Oberkiefers exakt eingestellt werden kann. Der Aufbau des Distraktors mit den Gewindespindeln ist sehr kompakt und von geringer Baugröße, so daß der Patient nur in einem Ausmaß, das unbedingt erforderlich ist, behindert wird. Darüberhinaus weist dieser Distraktor keine vorstehenden Teile auf, die innerhalb der Mundhöhle zu Verletzungen führen könnten, auch dann nicht, wenn die Verstellantriebe betätigt und die jeweiligen Teile 8, 18 und 17 in ihrem Abstand zueinander verändert wurden.

Da das anteriore Befestigungsteil 3 in der Kinnregion in der Ausführungsform des Distraktors 1 entsprechend der Figuren 1 und 2 angeordnet ist, kann der Zugang, um dieses Befestigungsteil 3 zu befestigen, über einen Schnitt von enoral in der beweglichen Schleimhaut im Vestibulum in einem Abstand von ungefähr 8 mm von der Grenze zur angewachsenen Gingiva in der Unterkieferfront erfolgen. In den Figuren ist die Durchtrittsstelle durch die Mukosa mit D bezeichnet. Weiterhin ist ersichtlich, daß aufgrund der Anordnung des Befestigungsteils 3 in dieser Kinnregion das foramen mentale Fm und damit der canalis mandibulae und der darin verlaufende nervus alveolaris inferior nicht tangiert werden.

Während anhand der Figuren 1 und 2 der rechtsseitige Distraktor 1 beschrieben worden ist, ist ersichtlich, daß der linksseitige Distraktor 1 einen entsprechenden, allerdings spiegelbildlichen Aufbau aufweist. Der linksseitige Distraktor 1 und der rechtsseitige Distraktor 1 können somit unabhängig voneinander in den zwei linearen Richtungen sowie um die Drehachse 7 verändert werden, falls eine beidseitige Korrektur des Unterkiefers notwendig ist.

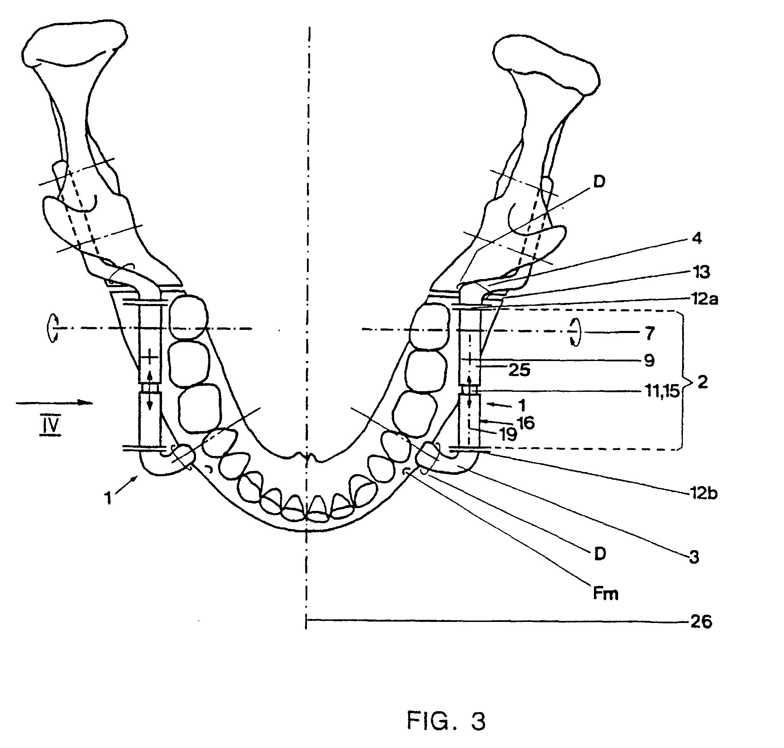

Die nachfolgend anhand der Figuren 3 bis 10 beschriebenen Ausführungsformen sind im Hinblick auf die Verstellmöglichkeiten in Richtung der Doppelpfeile 10 und 11 sowie um die Gelenkachse 7 mit derjenigen Ausführungsform, die vorstehend anhand der Figuren 1 und 2 beschrieben ist, vergleichbar. Daher werden auch, soweit die nachfolgenden Ausführungsformen Bauteile oder Baukomponenten aufweisen, die mit denjenigen der Ausführungsform der Figuren 1 und 2 vergleichbar sind, für solche Bauteile und Baukomponenten die entsprechenden Bezugszeichen verwendet, so daß die Ausführungen vorstehend in Bezug auf die Figuren 1 und 2 analog auf die weiteren Figuren in Bezug auf solche vergleichbaren Teile und deren Funktionsweisen übertragen werden können.

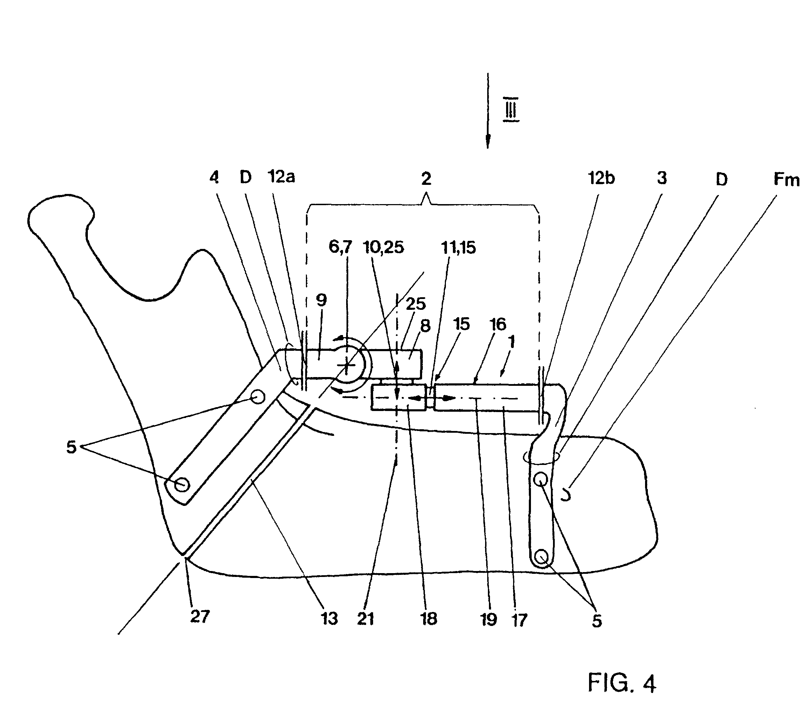

In der zweiten Ausführungsform, wie sie in den Figuren 3 und 4 dargestellt ist, entspricht, das Distraktionselement 2 des Distraktors, dem Aufbau der ersten Ausführungsform. Gleiches gilt für das dem ramus mandibulae zugeordneten, posterioren Befestigungsteil 4. Unterschiedlich ist dagegen das anteriore, dem corpus mandibulae zugeordnete Befestigungsteil 3, das mit seinem Befestigungsende, in Bezug auf das foramen mentale Fm, nicht im Kinnbereich verschraubt ist, sondern auf der zu der Osteotomie hinweisenden Seite, d.h. posterior des foramen mentale Fm. Bei diesem Distraktor erfolgt die Inzision von enoral im bukkalen Vestibulum in der beweglichen Schleimhaut, das Periost wird am corpus mandibulae partiell abgehoben, der nervus mentalis am foramen mentale Fm dargestellt und das anteriore Befestigungsteil 3, das hier etwa senkrecht zu dem Abschnitt 17 des freien Schenkels 16 verläuft und bis zum Unterkieferrand reicht, über die Löcher 5 mit Schrauben (posterior vom foramen mentale) fixiert. Die Löcher 5 sind weit voneinander beabstandet, so daß ein ausreichender Zwischenraum zwischen diesen Befestigungslöchern 5 verbleibt, so daß der nervus alveolaris inferior nicht während des Eingriffs tangiert wird. Diese zweite Ausführungsform stellt gegenüber der ersten Ausführungsform eine mögliche Alternative dar. Sie erfordert allerdings, daß für das transbukkale Einbringen der Schrauben meistens eine zweite kleine Hautinzision nötig ist und der chirurgische Zugang komplizierter sein kann. Zudem kann meist nur die caudale Befestigungsschraube bikortikal gesetzt werden, während die cranialen Schrauben des Befestigungsteils 3 wegen der Zahnwurzeln meist nur monokortikal verankert werden können. Allerdings hat diese Ausführungsform den Vorteil, daß die Kräfte aber mit einem Befestigungsteil 3, welches näher an der Osteotomie 13 liegt, mit kürzerem Hebelweg auf die Distraktion der Osteotomie übertragen werden können.

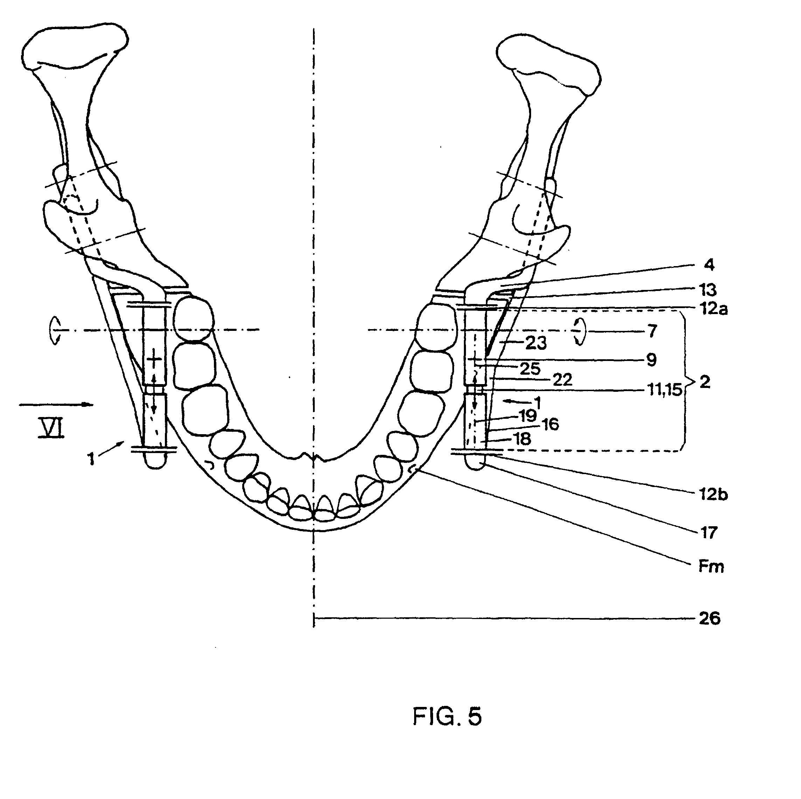

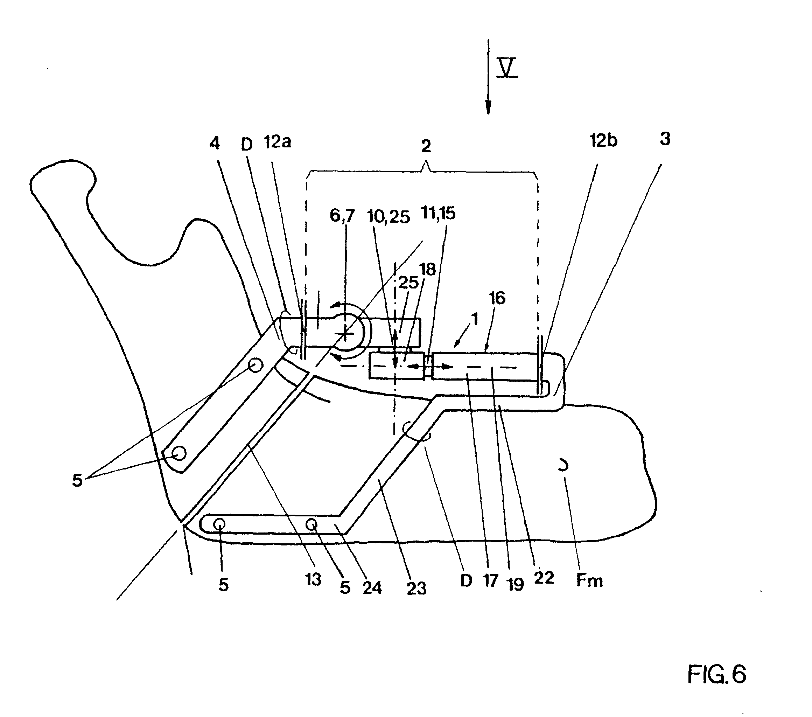

Die dritte Ausführungsform eines intraoralen Distraktors gemäß der Erfindung, wie er in den Figuren 5 und 6 gezeigt ist, entspricht wiederum in Bezug auf das Distraktionselement 2 und das posteriore Befestigungsteil 4 der ersten und der zweiten Ausführungsform (Figuren 1 bis 4). Im Unterschied zu der ersten und der zweiten Ausführungsform ist das anteriore Befestigungsteil 3 aus vier Abschnitten aufgebaut, d.h. aus dem Abschnitt 17 (vergleichbar mit den Abschnitten 17 der ersten und.der zweiten Ausführungsform), einem weiteren, parallel dazu verlaufenden Abschnitt 22, der sich in Richtung der Osteotomie 13 erstreckt, einem schräg verlaufenden Zwischenabschnitt 23 und den die Löcher 5 aufweisenden Befestigungsabschnitt, mit dem Bezugszeichen 24 bezeichnet. Dieser Befestigungsabschnitt 24 verläuft entlang des unteren Rands des corpus mandibulae und erstreckt sich bis fast zu der Osteotomie 13 hin. Wie insbesondere der Seitenansicht der Figur 6 zu entnehmen ist, verbleibt mit dieser Anordnung der Kinnbereich sowie der Bereich des Unterkiefers posterior des foramen mentale frei.

Diese Anordnung hat den Vorteil, daß die Distraktionskräfte ohne lange Hebelwege direkt auf die Osteotomie wirken, was vor allem im Vergleich zur ersten Ausführungsform bei der Distraktion in cranio-caudaler Richtung 10 ein Vorteil sein könnte. In der Regel können durch denselben transbukkalen Zugang, welcher für das Einbringen der Schrauben für das posteriore Befestigungsteil 4 benötigt wird, auch die Befestigungsschrauben am Abschnitt 24 des Befestigungsteils 3 eingebracht werden. Die Schrauben können in diesem Bereich meistens bikortikal gesetzt werden, da der nervus alveolaris inferior und die Wurzeln cranial von diesem Bereich liegen. Allerdings muß bei dieser Ausführungsform das Periost im Osteotomiebereich für das Befestigungsteil 3 mehr abgeschoben werden.

Es sei noch angemerkt, daß das Distraktionselement 2 in den vorstehend beschriebenen Ausführungsformen in Bezug auf die Schenkel 8, 9 sowie den freien Schenkel 16 des Befestigungsteils 3 mit den Abschnitten 17 und 18 parallel zu der Sagittalebene 26 verläuft bzw. ausgerichtet ist und die Gelenkachse 7 der Winkelverstellung senkrecht zur Sagittalebene ausgerichtet ist.

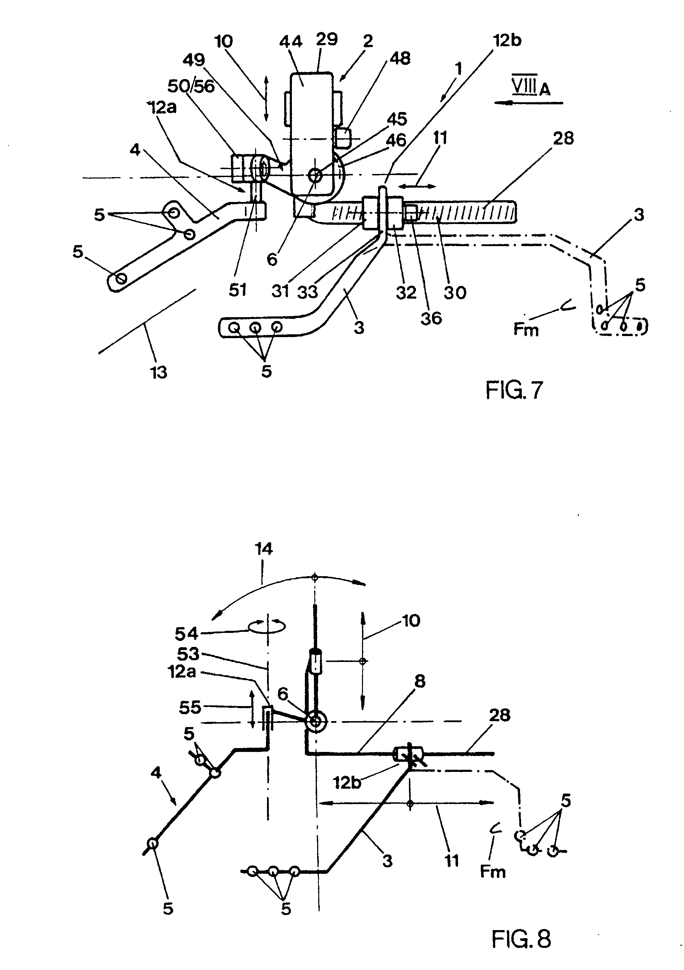

In den Figuren 7 bis 10 ist eine weitere, vierte Ausführungsform eines Distraktors 1 dargestellt, allerdings mit den einzelnen Bauteilen nicht an einem Unterkiefer befestigt, gezeigt, um die technischen Details übersichtlicher erläutern zu können. Auch bei diesem Distraktor 1 handelt es sich, wie die Figuren 7 und 8 zeigen, um einen Distraktor 1, der in Bezug auf drei Freiheitsgrade eine Verstellung des Kieferwinkels im Bereich einer Osteotomie 13, d.h. den Winkel zwischen corpus mandibulae und ramus mandibulae, ermöglicht. Zum einen ist wiederum das Gelenk 6 vorgesehen, zum anderen eine lineare Verschiebbarkeit in der posterio-anterioren Richtung, durch den Doppelpfeil 11 angedeutet, und eine Verschiebbarkeit in der cranio-caudalen Richtung, durch den Doppelpfeil 10 angedeutet, vorgesehen.

Die einzelnen Wirkungslinien des Distraktors 1 der Figur 7 sind schematisch in Figur 8, für eine bessere Übersicht, nochmals dargestellt.

Dieser Distraktor 1 der vierten Ausführungsform weist wiederum ein anteriores Befestigungsteil 3 mit Befestigungslöchern 5 auf, wobei das in durchgezogener Linie dargestellte Befestigungsteil einer Anordnung entspricht, wie sie anhand der dritten Ausführungsform beschrieben wurde und in den Figuren 5 und 6 (siehe insbesondere Figur 6) zu sehen ist, während das Befestigungsteil 3 in strichpunktierter Linie einem Aufbau entsprechend der ersten Ausführungsform (siehe Figuren 1 und 2) entspricht. Das posteriore Befestigungsteil 4 ist in seiner Anordnung vergleichbar mit den zuvor beschriebenen Ausführungsformen, allerdings sind in diesem posterioren Befestigungsteil 4 der Figur 7 drei Befestigungslöcher 5 vorgesehen, die in einer L-förmigen Anordnung verteilt sind, so daß die Kräfte, die auf diese Befestigungsstellen einwirken, besser verteilt werden, insbesondere dadurch, daß diese drei Löcher nicht auf einer geraden Linie liegen. Gleiches gilt auch für die gezeigte Anordnung der Befestigungslöcher 5 des distalen (anterioren) Befestigungsteils 3, die ebenfalls in der Ausführungsform der Figur 7, in einer L-Form verteilt angeordnet sind.

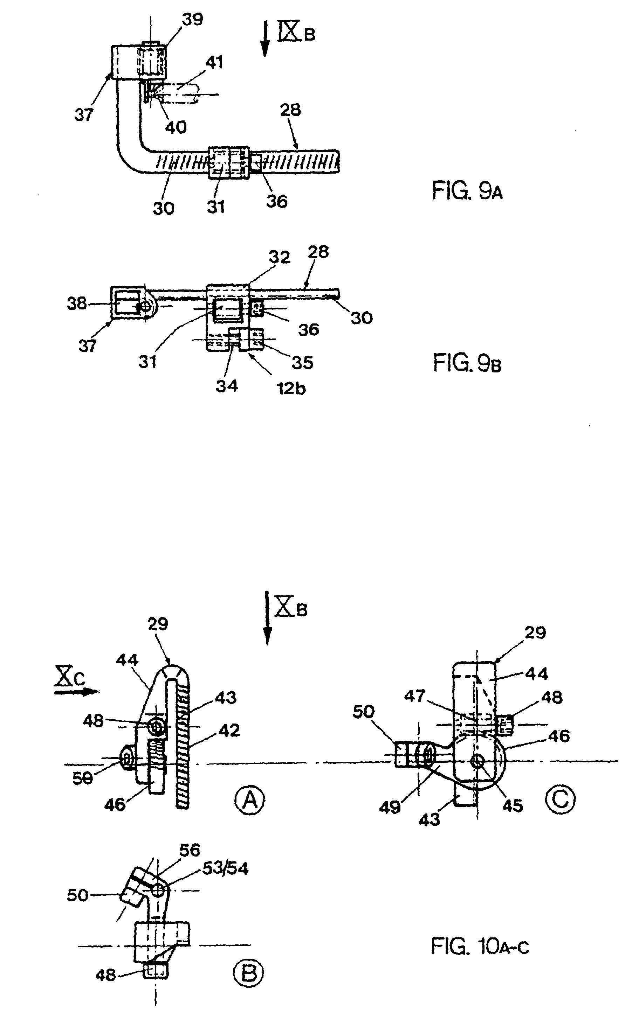

Das Distraktionselement 2 besteht aus zwei wesentlichen Komponenten, nämlich einem flachen, L-förmigen Bügelteil 28, das detaillierter, d.h. in zwei unterschiedlichen Ansichten, in den Figuren 9A und 9B zu sehen ist, wobei dieses Bügelteil vergleichbar mit dem Schenkel 8 des Distraktionselements, wie es in Figur 2 zu sehen ist, vergleichbar ist, und einem U-förmigen Bügelteil 29, vergleichbar mit dem Schenkel 9 der Ausführungsform gemäß Figur 2.

Das L-Bügelteil 28 ist als flaches, streifenförmiges Teil ausgebildet, mit einer Schrägverzahnung 30 auf seiner bukkalen Seite. In diese Schrägverzahnung greift ein Schneckenzahnrad 31 ein, das in einem Gehäuseteil 32 geführt ist, wobei wiederum das Gehäuseteil 32 an einem senkrecht stehenden Ende 33 des Befestigungsteils 3 lösbar in einem Klemmteil 34 gehalten ist; dieses Klemmteil 34 bildet gleichzeitig eine Trennstelle 12b, um das Distraktionselement 2 von dem Befestigungsteil 3 zu lösen. Für ein solches Lösen wird eine entsprechende Klemmschraube 35 gelöst, so daß das L-Bügelteil 28 nach oben von dem senkrecht stehenden Ende 33 abnehmbar ist. Das Schneckenzahnrad 31 verläuft mit seiner Drehachse parallel zu der Ebene der Schrägverzahnung 30 und ist von der Vorderseite über einen Kopf 36, beispielsweise mit einem Inneninbus versehen, zugänglich, so daß das Schneckenzahnrad 31 entlang dem langen Schenkel des L-Bügelteils, d.h. entlang dessen Schrägverzahnung 30, linear verschoben werden kann. An dem kurzen, senkrecht nach oben stehenden Schenkel des Bügelteils 28 ist ein Verbindungsteil befestigt, das einen Schlitz 38 sowie ein parallel zu diesem Schenkel mit seiner Antriebsachse verlaufendes Schneckenzahnrad 39 trägt. Dieses Schneckenzahnrad 39 ist an seinem unteren Ende mit einem Kegelzahnrad 40 versehen, über das, mit einem geeigneten Werkzeug 41, wie es in Figur 9A angedeutet dargestellt ist, drehbar ist. Aufgrund dieser Anordnung ist das Schneckenzahnrad 39 ebenfalls von vorne (anterior - von vorne durch die Mundhöhle) aus betätigbar.

In den Schlitz 38 wird der eine Schenkel des U-Bügels 29 (siehe Figur 10A) eingesteckt, so daß dessen seitliche Schrägverzahnung 43 mit dem Schneckenzahnrad 39 in Eingriff gelangt. Unter Verdrehung des Schneckenzahnrads 39 kann der L-förmige Bügel nach oben und nach unten, und zwar in Richtung des Doppelpfeils 10 (Figuren 7 und 8) verschoben werden. Der andere Schenkel 44 des U-Bügels trägt die Achse 45 eines schräg verzahnten Stirnzahnrads 46, wobei in dieses schräg verzahnte Stirnzahnrad ein weiteres Schneckenzahnrad 47 eingreift, dessen Achse wiederum so ausgerichtet ist, daß sie von der Vorderseite aus über einen Kopf 48 verdreht werden kann. Unter Verdrehung dieses Kopfes 48 wird das schräg verzahnte Stirnzahnrad 46 um seine Achse 45 gedreht, so daß ein darin angeordneter, sich nach hinten erstreckender (siehe Figur 7) Hebelarm 49 verschwenkt wird. Dieser Hebelarm 49 ist über eine Klemmhülse 56 mittels einer Klemmschraube 50 an einem Stift 51 festklemmbar, der an dem freien Ende des Befestigungsteils 4 angeordnet ist. Hierdurch wird eine zweite Trennstelle 12a gebildet. Wie ersichtlich ist, kann das Distraktionselement 2, wie es in seinen Einzelteilen in den Figuren 9 und 10 dargestellt ist, über die hintere Trennstelle 12a zwischen der Klemmschraube 50 und dem Stift 51 und der vorderen Klemmstelle im Bereich des Klemmteils 34 und der Klemmschraube 35 jederzeit von den Befestigungsteilen 3 und 4 lösbar, so daß dieses Distraktionselement 2 ausgetauscht werden kann, beispielsweise zu Wartungszwekken, oder um es gegen veränderten Gegebenheiten anzupassen. Weiterhin kann mittels der Rundstab-Führung 51 und der darauf aufgesetzten Klemmhülse 56 eine Verschiebung in Richtung des Doppelpfeils 55 erfolgen, d.h. die Hülse kann in der Höhe unterschiedlich an der Rundstab-Führung 51 verschoben und fixiert werden. Außerdem ist eine Drehung um die durch die Rundstab-Führung 51 gebildete Achse 53 in Richtung des Doppelpfeils 54 (siehe Figur 8) möglich, um auch so eine Anpassung, den Gegebenheiten entsprechend, vornehmen zu können. Weiterhin hat dieser Distraktor 1 auch den Vorteil, daß alle Verstellungen von der Vorderseite aus vorgenommen werden können, da alle Verstellteile gut von der Vorderseite aus mit einem Werkzeug zugänglich sind.

Es ist noch darauf hinzuweisen, daß die Darstellung der Figur 8 etwa maßstabsgerecht ist, so daß die Verhältnisse der Hebelarme sowie die Anlenkpunkte und Verbindungsstellen aus dieser Figur annähernd abgegriffen werden können.

## Patentansprüche

1. Intraoraler Distraktor für Kallusdistraktion im Unterkiefer eines Menschen mit einem proximalen (posterioren) und einem distalen (anterioren) Befestigungsteil (3, 4) und mit einem ersten Verstellantrieb zur linearen Veränderung des Abstands zwischen den beiden Befestigungsteilen (3, 4) zur Verlängerung des corpus mandibulae oder des ramus mandibulae im Bereich einer Osteotomie, wobei das eine Befestigungsteil (4) dem ramus mandibulae zugeordnet ist und das andere Befestigungsteil (3) dem corpus mandibulae zugeordnet ist
**dadurch gekennzeichnet,**
**daß** ein Distraktionselement (2) mit einem Gelenk (6) vorgesehen ist, dessen Gelenkachse (7) so orientiert ist, daß es eine Veränderung der winkelmäßigen Zuordnung von corpus mandibulae und ramus mandibulae zueinander im Bereich der Osteotomie (13) ermöglicht, und wobei das Distraktionselement (2) derart mit den Befestigungsteilen (3, 4) verbunden ist, daß es außerhalb des Gewebes im cavum oris und bukkal der Zahnreihe im Vestibulum liegt.

2. Intraoraler Distraktor nach Anspruch 1, **dadurch gekennzeichnet, daß** der erste Verstellantrieb (15) Teil des Distraktionselements (2) ist.

3. Intraoraler Distraktor nach Anspruch 1 oder Anspruch 2, **dadurch gekennzeichnet, daß** das Distraktionselement (2) zwischen den Befestigungsteilen (3, 4) lösbar im Bereich von Trennstellen (12a, 12b) gehalten ist.

4. Intraoraler Distraktor nach einem der Ansprüche 1 bis 3, **dadurch gekennzeichnet, daß** ein zweiter, linearer Verstellantrieb (25) vorgesehen ist für eine lineare Veränderung des Abstands zwischen den beiden Befestigungsteilen (3, 4) in einer Richtung mit einer Komponenten senkrecht zu der linearen Veränderung des ersten Versteilantriebs (15).

5. Intraoraler Distraktor nach Anspruch 1 oder Anspruch 4, **dadurch gekennzeichnet, daß** der zweite Verstellantrieb (25) Teil des Distraktionselements (2) ist.

6. Intraoraler Distraktor nach Anspruch 4 oder Anspruch 5, **dadurch gekennzeichnet, daß** der erste Verstellantrieb (15) eine Verlängerung des corpus mandibulae vornimmt und der zweite Verstellantrieb (25) eine Verlängerung des ramus mandibulae vornimmt.

7. Intraoraler Distraktor nach einem der Ansprüche 1 bis 6, **dadurch gekennzeichnet, daß** das Distraktionselement (2) einen Schenkel (8, 9), der dem dem ramus mandibulae zugeordneten Befestigungsteil (4) zugeordnetet ist, und einen Schenkel (17, 18), der dem dem corpus mandibulae zugeordneten Befestigungsteil (3) zugeordnet ist, aufweist.

8. Intraoraler Distraktor nach Anspruch 7, **dadurch gekennzeichnet, daß** sich die beiden Schenkel (8, 18) mit ihren freien Enden in Richtung der Erstreckung des corpus mandibulae gesehen überlappen.

9. Intraoraler Distraktor nach Anspruch 8, **dadurch gekennzeichnet, daß** die beiden einander überlappenden Enden (8, 18) einen Teil des zweiten Verstellantriebs (25) bilden derart, daß die beiden Enden in ihrem Abstand zueinander veränderbar sind.

10. Intraoraler Distraktor nach Anspruch 9, **dadurch gekennzeichnet, daß** der zweite Verstellantrieb (25) zur Abstandsveränderung eine Gewindespindel (21) umfaßt.

11. Intraoraler Distraktor nach Anspruch 7, **dadurch gekennzeichnet, daß** der den corpus mandibulae verlängernde erste Verstellantrieb (15) dem Schenkel (17, 18) zugeordnet ist, der Teil (17) des dem corpus mandibulae zugeordneten Befestigungsteils (3) ist.

12. Intraoraler Distraktor nach Anspruch 11, **dadurch gekennzeichnet, daß** der Schenkel (16) unterteilt ist und daß die beiden Schenkelteile (17, 18) mittels einer darin geführten Drehwelle (19) verstellbar verbunden sind.

13. Intraoraler Distraktor nach Anspruch 12, **dadurch gekennzeichnet, daß** die Drehwelle (19) über das Ende des Schenkels (17) zur Verstellung zugänglich ist, das mit dem Befestigungsteil (3) verbunden ist.

14. Intraoraler Distraktor nach Anspruch 1, **dadurch gekennzeichnet, daß** das Gelenk (6) Teil des dem ramus mandibulae zugeordneten Befestigungsteils (4) ist.

15. Intraoraler Distraktor nach einem der Ansprüche 1 bis 14, **dadurch gekennzeichnet, daß** das dem ramus mandibulae zugeordnete Befestigungsteil (4) auf der bukkalen bzw. lateralen Seite des ramus mandibulae befestigbar ist.

16. Intraoraler Distraktor nach Anspruch 1 und/oder Anspruch 4, **dadurch gekennzeichnet, daß** die Richtung(en) der linearen Veränderung(en) (11) parallel zu der Sagittalebene (26) ausgerichtet ist/sind.

17. Intraoraler Distraktor nach Anspruch 16, **dadurch gekennzeichnet, daß** die Gelenkachse (7) senkrecht zur Sagittalebene (26) ausgerichtet ist.

18. Intraoraler Distraktor nach Anspruch 1 und/oder Anspruch 4, **dadurch gekennzeichnet, daß** der erste und/oder der zweite Verstellantrieb (15, 25) durch eine Zahnstange (28, 30; 42, 43) und ein darin eingreifendes Schneckenzahnrad (31; 39) gebildet ist/sind.

19. Intraoraler Distraktor nach Anspruch 1, **dadurch gekennzeichnet, daß** das Gelenk (6) für die Veränderung der winkelmäßigen Zuordnung von ramus mandibulae und corpus mandibulae mittels eines Zahnradsegments (46) mit Schrägverzahnung und eines darin eingreifenden Schneckenzahnrads (47) erfolgt.

20. Intraoraler Distraktor nach Anspruch 19, **dadurch gekennzeichnet, daß** das Zahnradsegment (46) an dem dem ramus mandibulae zugeordneten Befestigungsteil (4) angeordnet ist und das Schneckenzahnrad (47) an dem dem corpus mandibulae zugeordneten Befestigungsteil (3) angeordnet ist.

21. Intraoraler Distraktor nach Anspruch 3, **dadurch gekennzeichnet, daß** die Trennstellen (12a, 12b) zwischen dem Distraktionselement (2) und dem jeweiligen Befestigungsteil (3, 4) jeweils durch eine lineare Führung gebildet ist, wobei die Führungsrichtung annähernd parallel zur Sagittalebene (26) etwa senkrecht zur Okklusionsebene des Unterkiefers verläuft.

22. Intraoraler Distraktor nach Anspruch 21, **dadurch gekennzeichnet, daß** die lineare Führung an der Trennstelle (12b) zu dem corpus mandibulae hin durch eine in eine Nut-Führung eingreifende Lasche gebildet ist.

23. Intraoraler Distraktor nach Anspruch 21, **dadurch gekennzeichnet, daß** die lineare Führung an der Trennstelle (12a) zu dem ramus mandibulae hin durch eine auf eine Rundstab-Führung (51) aufgesetzte Hülse (56) oder Klaue gebildet ist, wobei die Rundststab-Führung (51) Teil des Befestigungsteils (4) ist.

24. Intraoraler Distraktor nach Anspruch 23, **dadurch gekennzeichnet, daß** das Distraktionselement (2) durch Verschwenkung um die Achse (53) der Rundstab-Führung parallel zu der Sagittalebene (26) ausrichtbar ist.

25. Intraoraler Distraktor nach Anspruch 23 oder 24, **dadurch gekennzeichnet, daß** die Hülse (56) entlang der Rundstab-Führung (51) in Richtung der Achse (53) verstellbar (50) und fixierbar ist.

## Claims

1. An intraoral distractor for callus distraction in the human mandible with a proximal (posterior) and a distal (anterior) fixation unit (3, 4) and with a first displacement traction for the linear modification of the distance between the two fixation units (3, 4), in order to lengthen the corpus mandibulae or the ramus mandibulae in the area of the osteotomy, wherein the one fixation unit (4) is connected to the ramus mandibulae and wherein the other fixation unit (3) is connected to the corpus mandibulae,
**characterized by**
a distraction element (2) with a joint (6) of which the joint axis (7) is oriented such that it allows a modification of the angle between corpus mandibulae and ramus mandibulae relative to each other in the area of the osteotomy (13 and wherein the distraction element (2) is connected to the fixation units (3, 4) in such a way that it is positioned outside of the tissues within the oral cavity and buccal to the dentition in the vestibulum.

2. Intraoral distractor of claim 1, **characterized in that** the first displacement traction (15) is part of said distraction element (2).

3. Intraoral distractor of claim 1 or claim 2, **characterized in that** said distraction element (2) is held between said fixation units (3, 4) in a way that it can be loosened and removed by the help of interfaces (12A, 12B).

4. Intraoral distractor according to any one of claims 1 to 3, **characterized in that** second, linear displacement traction (25) is integrated in order to enable a linear change of the distance between said fixation units (3, 4) in a direction with a component orthogonal to the linear displacement achieved with said first displacement traction (15).

5. Intraoral distractor according to claim 1 or claim 4, **characterized in that** said second displacement traction (25) is part of said distraction element (2).

6. Intraoral distractor according to claim 4 or claim 5, **characterized in that** said first displacement traction (15) lengthens the corpus mandibulae and said second displacement traction (25) lengthens the ramus mandibulae.

7. Intraoral distractor according to any one of the claims 1 to 6, **characterized in that** said distraction element (2) has a leg (8,9), which is related to said fixation unit (4) connected to the ramus mandibulae, and a leg (17, 18) which is related to said fixation unit (3) connected to the corpus mandibulae.

8. Intraoral distractor according to claim 7, **characterized in that** the two legs (8, 18) are overlapping in direction of the length axis of the corpus mandibulae with their two ends.

9. Intraoral distractor according to claim 8, **characterized in that** said two overlapping ends (8, 18) form a part of a second displacement traction (25) such that the two ends can be modified in their distance to each other.

10. Intraoral distractor according to claim 9, **characterized in that** said second displacement traction (25) includes a threaded rod (21) in order to change the distance.

11. Intraoral distractor according to claim 7, **characterized in that** the first displacement traction (15) lengthening the corpus mandibulae is related to the branch (17, 18) which makes part (17) of the fixation unit (3) connected to the corpus mandibulae.

12. Intraoral distractor according to claim 11, **characterized in that** the leg (16) is segmented and the two segments (17, 18) of the leg are connected in an adjustable way by a threaded rod (19) guided within it.

13. Intraoral distractor according to claim 12, **characterized in that** the threaded rod (19) is accessible via the end of leg (17) which is connected to the fixation unit (3), in order to adjust it.

14. Intraoral distractor according to claim 1, **characterized in that** the joint (6) is part of the fixation unit (4) connected to the ramus mandibulae.

15. Intraoral distractor according to any one of claims 1 to 14, **characterized in that** the fixation unit (4) connected to the ramus mandibulae is fixated on the buccal respectively lateral side of the ramus mandibulae.

16. Intraoral distractor according to claim 1 and/or claim 4, **characterized in that** the direction(s) of the linear displacement(s) (11) are parallel to the sagittal plane (26).

17. Intraoral distractor according to claim 16, **characterized in that** the joint axis (7) is orthogonal to the sagittal plane (26).

18. Intraoral distractor according to claim 1 and/or claim 4, **characterized in that** the first and/or the second displacement traction (15,25) are formed by a toothed rack (28, 30; 42, 43) and an engaging worm gear (31; 39).

19. Intraoral distractor according to claim 1, **characterized in that** the joint (6) is rotated by a segment of a gear (46) with oblique toothing and an engaging worm gear (47), in order to change the relative angle between ramus mandibulae and corpus mandibulae.

20. Intraoral distractor according to claim 19, **characterized in that** the gear segment (46) is attached to the fixation unit (4) connected to the ramus mandibulae and wherein the worm gear (47) is attached to the fixation unit (3) connected to the corpus mandibulae.

21. Intraoral distractor according to claim 3, **characterized in that** the interfaces (12A, 12B) between the distraction element 2 and the corresponding fixation unit (3, 4) are each built by a linear guidance where the direction of guidance is about parallel to the sagittal plane (26) and about orthogonal to the occlusal plane of the mandible.

22. Intraoral distractor according to claim 21, **characterized in that** the linear guidance at the interface (12b) to the corpus mandibulae is formed by a butt strap reaching into a nut.

23. Intraoral distractor according to claim 21, **characterized in that** the linear guidance at the interface (12A) at the ramus mandibulae is formed by a sleeve or a claw (56) mounted on a rod guidance (51) which is part of the fixation unit (4).

24. Intraoral distractor according to claim 23, **characterized in that** the distraction element (2) can be arranged parallel to the sagittal plane (26) by turning about the axis (53) of the rod guidance.

25. Intraoral distractor according to claim 23 or claim 24, **characterized in that** the sleeve (56) can be moved (50) along the rod guidance (51) in direction of the axis (53) and can be fixated.

## Revendications

1. Distracteur intrabuccal pour la distraction du cal dans le maxillaire inférieur d'un humain comprenant une partie de fixation proximale (postérieure) et distale (antérieure) (3, 4) et un premier entraînement de déplacement pour la modification linéaire de la distance entre les deux parties de fixation (3, 4 pour l'allongement du ramus de la mandibule ou du corps de la mandibule dans la zone d'une ostéotomie, l'une des pièces de fixation (4) étant associée au ramus de la mandibule et l'autre pièce de fixation (3) étant associée au corps de la mandibule, **caractérisé**
**en ce qu'**un élément de distraction (2) est prévu avec une articulation (6) dont l'axe d'articulation (7) est réorienté de sorte que cela permet une modification de l'association angulaire du corps de mandibule et de la branche de mandibule l'un par rapport à l'autre dans le domina de l'ostéotomie (13) et l'élément de distraction (2) étant relié aux parties de fixation (3, 4) de sorte qu'il se trouve dans le vestibule à l'extérieur du tissu dans la cavité buccale (oris) de la rangée de dents.

2. Distracteur intrabuccal selon la revendication 1, que le premier entraînement de déplacement (15) fait partie de l'élément de distraction (2).

3. Distracteur intrabuccal selon la revendication 1 ou la revendication 2, **caractérisé en ce que** l'élément de distraction (2) est maintenu entre les parties de fixation (3, 4) de manière amovible à proximité de points de séparation (12a, 12b).

4. Distracteur inbrabuccal selon l'une des revendications 1 à 3, **caractérisé en ce qu'**un second entraînement de déplacement linéaire (25) est prévu pour une modification linéaire de la distance entre les deux pièces de fixation (3, 4) dans une direction avec un composant perpendiculairement à la modification linéaire du premier entraînement de déplacement (15.

5. Distracteur intrabuccal selon la revendication 1 ou la revendication 4, **caractérisé en ce que** le second entraînement de déplacement (25) fait partie de l'élément de distraction (2).

6. Distracteur intrabuccal selon la revendication 4 ou la revendication 5, **caractérisé en ce que** le premier entraînement de déplacement (15) effectue un allongement du corps de la mandibule et le second entraînement de déplacement (25), un allongement du ramus de la mandibule.

7. Distracteur intrabuccal selon l'une des revendications 1 à 6, **caractérisé en ce que** l'élément de distraction (2) présente une branche (8, 9) qui est associée à la partie de fixation (4) associée au ramus de mandibule et une branche (17, 18) associée à la partie de fixation (3) associée au corps de la mandibule.

8. Distracteur intrabuccal selon la revendication 7, **caractérisé en ce que** les deux branches (8, 18) se chevauchent par leurs extrémités en direction de l'extension du corps de mandibule.

9. Distracteur intrabuccal selon la revendication 8, **caractérisé en ce que** les deux extrémités se chevauchant (8, 18) forment une partie du second entraînement de déplacement (25) de sorte que la distance entre les deux extrémités est modifiable.

10. Distracteur intrabuccal selon la revendication 9, **caractérisé en ce que** le second entraînement de déplacement (25) comprend une tige filetée (21) pour modifier la distance.

11. Distracteur intrabuccal selon la revendication 8, **caractérisé en ce que** le premier entraînement de déplacement (15) allongeant le corps de la mandibule est associé à la branche (17, 18), branche qui fait partie (17) de la partie de fixation (3) associée au corps de mandibule.

12. Distracteur intrabuccal selon la revendication 11, **caractérisé en ce que** la branche (16) est divisée et **en ce que** les deux parties de branche (17, 18) sont reliées de manière réglable au moyen d'un arbre rotatif (19) guidé à l'intérieur.

13. Distracteur intrabuccal selon la revendication 12, **caractérisé en ce que** l'arbre rotatif (19) est accessible pour le déplacement par l'extrémité de la branche (17) qui est reliée à la partie de fixation (3).

14. Distracteur intrabuccal selon la revendication 1, **caractérisé en ce que** l'articulation (6) fait partie de la partie de fixation (4) associée au ramus de la mandibule.

15. Distracteur intrabuccal selon l'une des revendications 1 à 14, **caractérisé en ce que** la partie de fixation (4) associée du ramus de la mandibule peut être fixée sur le côté buccal respectivement latéral du ramus mandibulaire.

16. Distracteur intrabuccal selon la revendication 1 et/ou la revendication 4, **caractérisé en ce que** la (les) direction de la/des modification(s) linéaire(s) (11) est/sont parallèle(s) au plan sagittal (26).

17. Distracteur intrabuccal selon la revendication 16, **caractérisé en ce que** l'axe d'articulation (7) est orienté perpendiculairement au plan sagittal (26).

18. Distracteur intrabuccal selon la revendication 1 et/ou la revendication 4, **caractérisé en ce que** le premier et/ou le second entraînement de déplacement (15, 25) est/sont formé(s) par une crémaillère (28, 30; 42, 43) et une roue dentée à vis sans fin entrant en prise (31; 39).

19. Distracteur intrabuccal selon la revendication 1, **caractérisé en ce que** l'articulation (6) prévue pour la modification de l'association angulaire du ramus mandibulaire et du corps mandibulaire s'effectue par un segment de roue dentée (46) avec une denture inclinée et une roue dentée à vis sans fin (47) s'y engageant.

20. Distracteur intrabuccal selon la revendication 19, **caractérisé en ce que** le segment de roue dentée (46) est disposé sur la partie de fixation (4) associée au ramus mandibulaire et la roue dentée à vis sans fin (47) est disposée sur la partie de fixation (3) associée au corps de mandibule.

21. Distracteur intrabuccal selon la revendication 3, **caractérisé en ce que** les points de séparation (12a, 12b) sont formés entre l'élément de distraction (2) et la partie de fixation respective (3, 4) respectivement par un guide linéaire, le sens de guidage étant sensiblement parallèle au plan sagittal (26) sensiblement perpendiculairement au plan d'occlusion du maxillaire inférieur.

22. Distracteur intrabuccal selon la revendication 21, **caractérisé en ce que** le guide linéaire est formé au point de séparation (12b) en direction du corps de mandibule par une languette s'engageant dans une rainure de guidage.

23. Distracteur intrabuccal selon la revendication 21, **caractérisé en ce que** le guide linéaire est formé au point de séparation (12a) en direction du ramus de mandibule par une gaine (56) ou pince placée sur un bâtonnet rond de guidage, le bâtonnet rond de guidage (51) faisant partie de la partie de fixation (4).

24. Distracteur intrabuccal selon la revendication 23, **caractérisé en ce que** l'élément de distraction (2) est orientable par pivotement autour de l'axe (53) du bâtonnet parallèlement au plan sagittal (26).

25. Distracteur intrabuccal selon la revendication 23 ou 24, **caractérisé en ce que** la douille (56) est réglable (50) et fixable le long du bâtonnet rond de guidage (51) en direction de l'axe (53).
